# EUROPEAN PATENT APPLICATION

(11) **EP 4 851 175 A1**
(43) Date of publication of application: **09.12.2026**
(21) Application number: 25749151.4
(22) Date of filing: 23.01.2025
(51) Int. Cl.: C07H 21/00, C07H 1/00, C12N 15/113

(54) **IMPROVED METHOD FOR OLIGONUCLEOTIDE SYNTHESIS**

(30) Priority: 02.02.2024 KR 20240016689; 20.12.2024 KR 20240192471
(71) Applicant: ST Pharm Co., Ltd., Siheung-si, Gyeonggi-do 15086 (KR)
(72) Inventor: KIM, Sung Won, Bucheon-si, Gyeonggi-do 14694 (KR); YUN, Ji Suk, Gwacheon-si, Gyeonggi-do 13838 (KR); LEE, Ju Young, Ansan-si, Gyeonggi-do 15459 (KR); BYUN, Myeong Seop, Ansan-si, Gyeonggi-do 15357 (KR); PARK, Kyu Nam, Ansan-si, Gyeonggi-do 15358 (KR)
(74) Representative: Vossius & Partner Patentanwälte Rechtsanwälte mbB
(86) International application number: PCT/KR2025/001279
(87) International publication number: WO 2025/165064

(57) **Abstract**

The present invention relates to an improved method for oligonucleotide synthesis. In the method for oligonucleotide synthesis according to the present invention, a synthesis cycle is performed at a temperature lower than room temperature, thereby suppressing side reactions occurring during the synthesis process and stably synthesizing oligonucleotides with higher purity.

## Description

### Technical Field

The present invention relates to an improved method for oligonucleotide synthesis.

### Background Art

The synthesis of oligonucleotides has established itself as an essential technology in the fields of life science and medicine. In particular, for oligonucleotides, which are widely used in gene research, diagnosis, therapeutic development, and genetic engineering, the accuracy and purity of synthesis are extremely important. If the quality of synthesized oligonucleotides is low, it may lead to errors in research and diagnostic results, and especially when used for medicinal purposes, it may result in reduced efficacy or side effects. For this reason, the development of high-purity and high-efficiency synthesis methods for oligonucleotides is emerging as an important task.

The phosphoramidite synthesis method has been established as the standard method for oligonucleotide synthesis since its introduction in the 1980s. This synthesis method links nucleoside precursors stepwise and boasts high precision, ensuring an accurate base sequence at each step. The phosphoamidite synthesis method proceeds by protecting the 5' end of the nucleoside while using the phosphoamidite group at the 3' end to induce bonding on a solid support. This has enabled large-scale synthesis and precise sequence control using automated instruments, and is demonstrating high utility in research and industrial settings.

However, conventional phosphoamidite synthesis methods have limited efficiency, and high-purity synthesis is difficult due to the potential for incomplete bonding or the generation of byproducts resulting from side reactions. Therefore, there is an increasing need for methods to minimize side reactions and enable stable synthesis in order to increase the purity and yield of oligonucleotides.

The inventors of this application have continuously conducted in-depth research to improve the efficiency and purity of the oligonucleotide synthesis process and have explored various approaches to overcome the limitations of existing synthesis methods. As a result, the inventors of the present application have improved upon the limitations of existing technology to suppress side reactions occurring during the synthesis process and have derived the present invention, which enables the stable synthesis of high-purity oligonucleotides.

### Disclosure of Invention

### Technical Goals

One aspect of the present invention provides a method for synthesizing an oligonucleotide on a solid support comprising the following steps (i) to (v), wherein one or more of the steps are performed at a temperature below room temperature (RT):
(i) removing a protecting group from a nucleoside or nucleotide having a protecting group on a hydroxyl group, thiol group, or amino group at the 5' or 3' position to form a deprotected nucleoside or nucleotide having a hydroxyl group, thiol group, or amino group at the 5' or 3' position;
(ii) a step of coupling a nucleoside phosphoamidite or nucleotide phosphoamidite having a protecting group on a hydroxyl group, thiol group, or amino group at the 5' or 3' position to a deprotected hydroxyl group, thiol group, or amino group at the 5' or 3' position;
(iii) a step of oxidizing the phosphite ester group generated through coupling to form a phosphate ester group, thiolating to form a phosphorothioate ester group, or imizing to form a phosphoramidate group;
(iv) removing the protecting group of a hydroxyl group, thiol group, or amino group at the 5' or 3' position of a coupled nucleoside phosphoamidite or nucleotide phosphoamidite to form a deprotected hydroxyl group, thiol group, or amino group at the 5' or 3' position;
(v) A step of synthesizing a desired oligonucleotide by repeating steps (ii) to (iv) one or more times.

One aspect provides a method for synthesizing an oligonucleotide in which the nucleoside or nucleotide having a protecting group on the hydroxyl group, thiol group, or amino group at the 3' position is an abasic nucleoside or nucleotide.

One aspect provides a method for synthesizing an oligonucleotide, wherein the temperature below the room temperature (RT) is less than 22°C.

### Technical Solutions

One aspect of the present invention provides a method for synthesizing an oligonucleotide on a solid support comprising the following steps (i) to (v), wherein one or more of the steps are performed at a temperature below room temperature (RT):.
(i) removing a protecting group from a nucleoside or nucleotide having a protecting group on a hydroxyl group, thiol group, or amino group at the 5' or 3' position to form a deprotected nucleoside or nucleotide having a hydroxyl group, thiol group, or amino group at the 5' or 3' position;
(ii) a step of coupling a nucleoside phosphoamidite or nucleotide phosphoamidite having a protecting group on a hydroxyl group, thiol group, or amino group at the 5' or 3' position to a deprotected hydroxyl group, thiol group, or amino group at the 5' or 3' position;
(iii) a step of oxidizing the phosphite ester group generated through coupling to form a phosphate ester group, thiolating to form a phosphorothioate ester group, or imizing to form a phosphoramidate group;
(iv) removing the protecting group of a hydroxyl group, thiol group, or amino group at the 5' or 3' position of a coupled nucleoside phosphoamidite or nucleotide phosphoamidite to form a deprotected hydroxyl group, thiol group, or amino group at the 5' or 3' position;
(v) A step of synthesizing a desired oligonucleotide by repeating steps (ii) to (iv) one or more times.

The oligonucleotide synthesis method according to the present invention may further include the step of capping a deprotected hydroxyl group, a thiol group, or an amino group that has not undergone a coupling reaction.

In one embodiment, the capping step may be additionally included in the oligonucleotide synthesis method according to the present invention when the step (iii) is a step of oxidizing a phosphite ester group generated through coupling to form a phosphate ester group.

In the method for synthesizing oligonucleotides according to the present invention, when steps (ii) to (iv) are repeated one or more times, in one or more of the repeated cycles, one or more of steps (ii) to (iv) may be performed at a temperature below room temperature. Specifically, one or more of the repeated cycles include all possible cycles, such as one or more, two or more, three or more, etc., and may include cycles selected discontinuously. Additionally, specifically, in one or more of the iterations, steps (ii), (iii), (iv), (ii) and (iii), (ii) and (iv), (iii) and (iv), or steps (ii), (iii) and (iv) may be performed at a temperature below room temperature.

In the method for synthesizing oligonucleotides according to the present invention, the last step of the last iteration may be step (iii) or (iv).

The method for synthesizing an oligonucleotide according to the present invention may further include a step of removing a phosphorus protecting group after step (v).

In the method for synthesizing oligonucleotides according to the present invention, the nucleoside may refer to a compound comprising a sugar group covalently bonded to a nucleobase. Nucleobases are used interchangeably with bases. Each of the above nucleosides may contain a nucleobase.

In the present invention, the terms "nucleobase" or "base" mean purines and pyrimidines, such as adenine, thymine, cytosine, and guanine which are nucleobases of DNA, adenine, uracil, cytosine, and guanine which are nucleobases of RNA, and non-DNA/RNA nucleobases. And, non-DNA/RNA nucleobases may be nitrogenous bases, for example, including 5-methylcytosine (MeC), isocytosine, pseudoisocytosine, 5-bromouracil, 5-propynyluracil, 5-propynyl-6-fluorouracil, 5-methylthiazoluracil, 6-aminopurine, 2-aminopurine, inosine, 2,6-diaminopurine, 7-propin-7-diazaadenine, 7-propin-7-diazaguanine, and 2-chloro-6-aminopurine.

A nucleoside according to one embodiment may be a naturally occurring nucleoside in which the sugar group is deoxyribose or ribose and the nucleobase is adenine, thymine, uracil, cytosine, or guanine, or a modified nucleoside.

The above modified nucleoside may have a modified sugar group. Specifically, modification of the sugar group may include replacing the ribose ring with a hexose, cyclopentyl, or cyclohexyl ring, replacing the D-ribose or D-deoxyribose ring of naturally occurring nucleic acid with an L-ribose or L-deoxyribose ring, or replacing the β-anomer of naturally occurring nucleic acid with an α-anomer.

The above modified nucleoside may have a modified nucleobase. The modified nucleus may be the above-mentioned non-DNA/RNA nucleus.

In one embodiment, the modified nucleoside may be a 2'-modified nucleoside or a 2'-substituted nucleoside. Specifically, the modified nucleoside may be a nucleoside modified or substituted with 2'-O-alkyl, 2'-O-methyl, 2'-O-ethyl, 2'-fluoro, 2'-O-methoxyethyl, 2'-amino, 2'-arabino-fluoro, 2'-O-aryl, 2'-O-benzyl, 2'-O-allyl, 2'-acetyl, 2'-aminomethyl, 2'-thio, or 2'-O-methyl-4-pyridine.

In one embodiment, the modified nucleoside may have a hydroxyl group (-OH) at the 5' or 3' position substituted with a thiol group (-SH) or an amino group (-NH2).

In one embodiment, the modified nucleoside may be an abasic nucleoside. An abasic nucleoside refers to a nucleoside that does not have a nucleobase attached. More specifically, it refers to a sugar group of a nucleoside or a sugar moiety of a nucleoside to which a nucleobase is not attached.

In one embodiment, the modified nucleoside may be an inverted nucleoside. An inverted nucleoside refers to a nucleoside that has a structure with a binding direction opposite to that of a normal nucleoside. For example, in a method for synthesizing oligonucleotides on a solid support, a normal nucleoside on the solid support is connected to the next unit through a functional group at the 5' position, but an inverted nucleoside is connected to the next unit through a functional group at the 3' position.

In one embodiment, the modified nucleoside may be an inverted abasic nucleoside.

In the method for synthesizing an oligonucleotide according to the present invention, the nucleotide may refer to a compound in which a sugar group, a nucleobase, and a phosphate group are combined (e.g., nucleic acid, DNA, RNA, or an analog thereof). Nuclear bases are used interchangeably with bases. Each of the above nucleotides may contain a nucleobase. Nucleobases or bases are as described above.

In one embodiment, the nucleotide may be a naturally occurring nucleotide in which the sugar group is deoxyribose or ribose and the nucleobase is adenine, thymine, uracil, cytosine, or guanine, for example, adenosine triphosphate, adenosine diphosphate, adenosine monophosphate, thymidine triphosphate, thymidine diphosphate, thymidine monophosphate, guanosine triphosphate, guanosine diphosphate, guanosine monophosphate, cytidine triphosphate, cytidine diphosphate, cytidine monophosphate, uridine triphosphate, uridine diphosphate, uridine monophosphate.

In addition, the above nucleotide may be a modified nucleotide.

The above modified nucleotide may have a modified sugar group. The variations of the group are as described above.

The above modified nucleotide may be a modified nucleobase. The modified nucleobase is as described above.

The above modified nucleotide may have a modified phosphate group. The modified phosphate group may be phosphorothioate, phosphodithioate, methylphosphonate, phosphate methyl, or phosphoramidate.

The above modified nucleotide may have a protecting group attached to a phosphorus residue. The above-mentioned protecting group may be a methyl group or a cyanoethyl group.

In one embodiment, the modified nucleotide may be a 2'-modified nucleotide or a 2'-substituted nucleotide. Specifically, the modified nucleotide may be a nucleotide modified or substituted with 2'-O-alkyl, 2'-O-methyl, 2'-O-ethyl, 2'-fluoro, 2'-O-methoxyethyl, 2'-amino, 2'-arabino-fluoro, 2'-O-aryl, 2'-O-benzyl, 2'-O-allyl, 2'-acetyl, 2'-aminomethyl, 2'-thio, or 2'-O-methyl-4-pyridine.

In one embodiment, the modified nucleotide may have a hydroxyl group (-OH) at the 5' or 3' position substituted with a thiol group (-SH) or an amino group (-NH2).

In one embodiment, the modified nucleotide may be an abasic nucleotide. An abasic nucleotide refers to a nucleotide that does not have a nucleobase attached. More specifically, it means the sugar group and phosphate group of a nucleotide, or the sugar and phosphate moiety of a nucleoside, to which a nucleobase is not attached.

In one embodiment, the modified nucleotide may be an inverted nucleotide. An inverted nucleotide refers to a nucleotide that has a structure in which the bonding direction is opposite to that of a normal nucleotide. For example, in a method for synthesizing oligonucleotides on a solid support, a normal nucleotide is connected to the next monomer through a functional group at the 5' position, but an inverted nucleotide is connected to the next monomer through a functional group at the 3' position.

In one embodiment, the modified nucleotide may be an inverted abasic nucleotide.

In one embodiment, the nucleotide may be a nucleotide, a di-nucleotide, a tri-nucleotide, or a nucleotide of an oligomer with more than one oligomer.

In the method for synthesizing an oligonucleotide according to the present invention, the nucleoside or nucleotide having a protecting group on a hydroxyl group, thiol group, or amino group at the 5' or 3' position may be immobilized on a solid support.

In one embodiment, the solid support may be resin, glass, cellulose, nylon, acrylamide gel, dextran, CPG (Controlled Pore Glass), polystyrene, TentaGel, silica-based support, trityl resin, amphorite resin, cellulose-based support, GMA (Glycidyl Methacrylate)-based support, or PEG-modified support. However, it is not limited to this.

In one embodiment, the nucleoside or nucleotide having a protecting group on the hydroxyl group, thiol group, or amino group at the 5' position may be fixed to a solid support through the 3' position or the 2' position, and the nucleoside or nucleotide having a protecting group on the hydroxyl group, thiol group, or amino group at the 3' position may be fixed to a solid support through the 5' position or the 2' position. The nucleoside or nucleotide having a protecting group on the hydroxyl group, thiol group, or amino group at the 3' position may be an abasic nucleoside or nucleotide, and may be an inverted abasic nucleoside or nucleotide.

In the method for synthesizing an oligonucleotide according to the present invention, the protecting group may be a known protecting group that protects a hydroxyl group, a thiol group, or an amino group at the 5' or 3' position. For example, the above protecting group is trityl (Trt), dimethoxytrityl (DMT), monomethoxytrityl (MMT), tert-butyldimethylsilyl (TBDMS), trimethylsilyl (TMS), t-butoxycarbonyl (Boc), methyl phosphodiester, benzoyl (Bz), trifluoroacetyl (TFA), benzyl (Bn), p-methoxytriphenylmethyl (pMT), mono-methoxyphosphodiester (MMP), fluorenyl methoxycarbonyl (Fmoc), tetrahydrofuran (THP), acetyl (Ac), mercapto-trityl, It may be a protecting group of 4-methoxycitrate (Mmt), thiopyridyl, benzylthio, fluorenylmethoxycarbonyl (Fmoc), carbamate (Cbz), trifluoroacetyl (TFA) or isobutyryl (iBu), phenoxyacetyl (PAC), or dimethylformamidine (dmf).

In the present invention, the term protector removal is used interchangeably with deprotection or protector detachment.

In one embodiment, the removal of the protector is possible under appropriate acidic or basic conditions. The above method for removing the protector includes a general protector removal protocol optimized for each protector, which is known in the industry.

That is, by removing the protecting group from a nucleoside or nucleotide having a protecting group on a hydroxyl group, thiol group, or amino group at the 5' or 3' position according to one embodiment, a deprotected nucleoside or nucleotide having a hydroxyl group, thiol group, or amino group at the 5' or 3' position can be formed.

In the method for synthesizing an oligonucleotide according to the present invention, through the coupling, a nucleoside phosphoamidite or a nucleotide phosphoamidite having a protecting group on the hydroxyl group, thiol group, or amino group at the 5' or 3' position can be connected to the deprotected hydroxyl group, thiol group, or amino group at the 5' or 3' position.

In the oligonucleotide synthesis method according to the present invention, the oxidation can increase stability by converting the phosphite ester group generated through coupling into a phosphate ester group. Such oxidation can be achieved using protocols known in the art, for example, iodine (I₂) solution or tert-butyl hydroperoxide (t-BuOOH), etc.

In the oligonucleotide synthesis method according to the present invention, the sulfide can increase stability by converting the phosphite ester group generated through coupling into a phosphorothioate ester group. Such sulfation can be achieved using protocols known in the art, for example, phenylacetyl disulfide (PADS), bis(oxadiazolyl)sulfide (BOBS), disulfide tetrazole (DDTT), or xanthan hydride.

In the oligonucleotide synthesis method according to the present invention, the iminization can increase stability by converting the phosphite ester group generated through coupling into a phosphoramidate group. Such immigration can be achieved using protocols known in the industry.

In the oligonucleotide synthesis method according to the present invention, the capping may serve to deactivate a deprotected hydroxyl group, thiol group, or amino group at the 5' or 3' position that has not undergone a coupling reaction so as not to participate in additional oligonucleotide synthesis reactions. Such capping can be achieved using protocols known in the art, for example, hydroxyl groups can be deactivated through an acetylation reaction using acetic anhydride (Ac₂O) and N-methylimidazole (NMI), thiol groups can be deactivated into thioester or methyl thio forms using methyl iodide (MeI) or acetyl disulfide, and amino groups can be deactivated through an acetylation or carbonylation reaction using acetic anhydride (Ac₂O) or t-butoxycarbonyl (t-Boc), etc.

In the method for synthesizing an oligonucleotide according to the present invention, the removal of the phosphorus protecting group may be the removal of the phosphorus protecting group of the backbond of the oligonucleotide. In one embodiment, the phosphorus protecting group may be a methyl group or a cyanoethyl group. The removal of phosphorus protecting groups can be achieved using protocols known in the art, and the methyl group can be removed using, for example, thiophenol or disodium 2-carbamoyl-2-cyanoethylene-1,1-dithioleate, and the cyanoethyl group can be removed using, for example, non-nucleophilic or hindered amines, such as diethylamine, tert-butylamine or 1,8-Diazabicycloundec-7-ene (DBU).

In the method for synthesizing an oligonucleotide according to the present invention, the oligonucleotide may refer to a compound having a structure in which nucleoside or nucleotide units are linked through phosphate esters, phosphorothioate esters, or phosphoramidate bonds. Nucleosides and nucleotides are as described above.

In one embodiment, the synthesized oligonucleotide may be 2 to 100 oligomers (2-100 mer), or more.

A method for synthesizing an oligonucleotide according to the present invention may further include, prior to step (i), a step of preparing a nucleoside or nucleotide that is immobilized on the surface of a solid support and has a protecting group on a hydroxyl group, a thiol group, or an amino group at the 5' or 3' position.

In one embodiment, the preparing step may include the step of immobilizing a nucleoside or nucleotide having a protecting group on a hydroxyl group, thiol group, or amino group at the 5' or 3' position on a solid support.

In one embodiment, the fixation may be such that the nucleoside or nucleotide is directly connected to the solid support or connected through a linker.

In one embodiment, the linker may be designed to attach a nucleoside or nucleotide to a solid support through various chemical bonds. The above linker is, for example, a universal linker, UnyLinker^{™}, a diacyl moiety, an aminoalkyl moiety, an alkyl moiety, an alkenyl moiety, a phosphate moiety, a succinyl moiety, an oxalyl moiety, a malonyl moiety, a glutaryl moiety, a trityl moiety, a polyethylene glycol (PEG) moiety, or -OC(O)CH2CH2C(O)O-, - NHC(O)CH2CH2C(O)NH-, -NHC(O)CH2CH2C(O)O-, -OC(O)CH2CH2C(O)NH-, -OC(O)CH2CH2C(O)S-, -NHC(O)CH2CH2C(O)S-, -OC(O)CH2C(O)O-, - NHC(O)CH2C(O)NH-, -NHC(O)CH2C(O)O-, -OC(O)CH2C(O)NH-, - OC(O)CH2C(O)S-, -NHC(O)CH2C(O)SH-, -OC(O)C(O)O-, -NHC(O) C(O)NH-, - NHC(O) C(O)O-, -OC(O)C(O)NH-, -OC(O)C(O)S-, -NHC(O) C(O)S-, -C(O)O-, - C(O)NH-, -C(O)S- -NH-, -O-, -S-, -S-S-, -C(N)H-O-, -NHC(O)O-, It may be a linker containing a moiety of -NHC(O)NH-, -OC(O)O-, -OPO3-, or a combination thereof. However, it is not limited to this.

The right end of the above moiety may represent a connection site with a nucleoside or nucleotide. The above moiety may include a spacer at the left end. The above spacers can be various spacers used in the industry for oligonucleotide synthesis, such as C1-30 alkyl, C2-30 alkenyl, and polyethylene glycol (PEG).

The above linker includes a spacer to secure a physical distance between the solid support and the nucleoside or nucleotide.

The above fixation method can be selectively adjusted according to specific synthesis conditions or goals and may include various binding methods that stabilize the binding between the solid support and the nucleotide or impart functionality. However, this fixing method is not limited to this.

The method for synthesizing an oligonucleotide according to the present invention may further include a step of recovering the synthesized oligonucleotide.

In one embodiment, the recovery step may mean detaching, separating, separating, cutting, or cleavaging the desired oligonucleotide from a solid support after it has been synthesized. Additionally, the above recovery may also include removing the protector. The above protector may mean a cyanoethyl protector of a phosphoamidite group and/or a protector of a base.

Specifically, the recovery step may mean removing the cyanoethyl protecting group of the phosphoamidite group of the synthesized oligonucleotide after the desired oligonucleotide is synthesized, desorbing the synthesized oligonucleotide from the solid support, and removing the protecting group of the base.

This recovery step can be achieved using protocols known in the industry. The above recovery can be achieved using reagents such as diethylamine, triethylamine, ammonium hydroxide, and methylamine. Specifically, diethylamine and triethylamine can be primarily used to remove the cyanoethyl protecting group of the phosphoamidite group, and ammonium hydroxide, methylamine, or a combination thereof can be primarily used to desorb oligonucleotides synthesized from a solid support and remove the protecting group of the base.

In a method for synthesizing an oligonucleotide according to the present invention, in step (i),

A nucleoside or nucleotide having a protecting group on a hydroxyl, thiol, or amino group at the 5' position is immobilized on the surface of a solid support through the 3' or 2' position;

A nucleoside or nucleotide having a protecting group on a hydroxyl, thiol, or amino group at the 3' position may be immobilized on the surface of a solid support through the 5' or 2' position.

In one embodiment, the nucleoside or nucleotide having a protecting group on a hydroxyl group, thiol group, or amino group at the 3' position in step (i) may be an abasic nucleoside or nucleotide. The above abasic nucleoside or nucleotide may be an inverted abasic nucleoside or nucleotide.

In one embodiment, the abasic nucleoside may be a compound represented by the following chemical formula I:

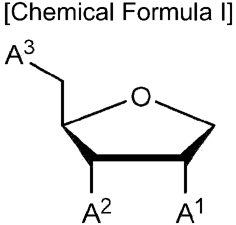

In the above formula I, A1 is -H, -OH, 2'-O-alkyl, 2'-O-methyl, 2'-O-ethyl, 2'-fluoro, 2'-O-methoxyethyl, 2'-amino, 2'-arabino-fluoro, 2'-O-aryl, 2'-O-benzyl, 2'-O-allyl, 2'-acetyl, 2'-aminomethyl, 2'-thio, or 2'-O-methyl-4-pyridine, and
A2 is -OH, -NH2, or -SH, and
A3 is an -OH, -NH2, -SH, -OPO3, -O(PO3)2, -O(PO3)3, or phosphoamidite functional group.

The above phosphomidite functional group is represented as - OP(OR1)NR22. In one embodiment, R1 and R2 are phosphoramidite substituents, and each independently may be -H, C1-7 alkyl group, C5-20 or C5-20 aryl group. In one embodiment, the phosphoamidite functional group may be -OP(OCH2CH3)N(CH3)2, -OP(OCH2CH3)N(i-Pr)2, or -OP(OCH2CH2CN)N(i-Pr)2. However, it is not limited to these.

In a method for synthesizing oligonucleotides according to the present invention,

The above-mentioned basic nucleoside may be an inverted basic nucleoside represented by the following formula I, immobilized on the surface of a solid support via a linker:

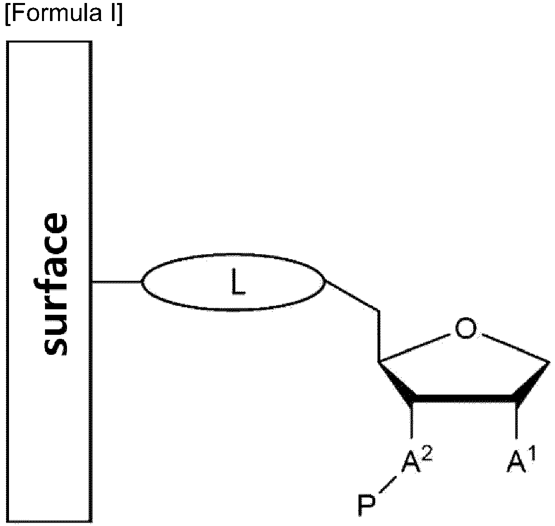

In the above equation, P is the protector and L is the linker, and
A1 is -H, -OH, 2'-O-alkyl, 2'-O-methyl, 2'-O-ethyl, 2'-fluoro, 2'-O-methoxyethyl, 2'-amino, 2'-arabino-fluoro, 2'-O-aryl, 2'-O-benzyl, 2'-O-allyl, 2'-acetyl, 2'-aminomethyl, 2'-thio, or 2'-O-methyl-4-pyridine, and
A2 is -O-, -NH-, or -S-.

The above protecting group P may be a known protecting group that protects a hydroxyl group, a thiol group, or an amino group. For example, the above protecting group is trityl (Trt), dimethoxytrityl (DMT), monomethoxytrityl (MMT), tert-butyldimethylsilyl (TBDMS), trimethylsilyl (TMS), t-butoxycarbonyl (Boc), methyl phosphodiester, benzoyl (Bz), trifluoroacetyl (TFA), benzyl (Bn), p-methoxytriphenylmethyl (pMT), mono-methoxyphosphodiester (MMP), fluorenyl methoxycarbonyl (Fmoc), tetrahydrofuran (THP), acetyl (Ac), mercapto-trityl, It may be a protecting group of 4-methoxycitrate (Mmt), thiopyridyl, benzylthio, fluorenylmethoxycarbonyl (Fmoc), carbamate (Cbz), trifluoroacetyl (TFA) or isobutyryl (iBu), phenoxyacetyl (PAC), or dimethylformamidine (dmf).

The above linker can be designed to attach a compound of Formula I to a solid support through various chemical bonds. The above linker is, for example, a universal linker, UnyLinker^{™}, a diacyl moiety, an aminoalkyl moiety, an alkyl moiety, an alkenyl moiety, a phosphate moiety, a succinyl moiety, an oxalyl moiety, a malonyl moiety, a glutaryl moiety, a trityl moiety, a polyethylene glycol (PEG) moiety, or -OC(O)CH2CH2C(O)O-, -NHC(O)CH2CH2C(O)NH-, - NHC(O)CH2CH2C(O)O-, -OC(O)CH2CH2C(O)NH-, -OC(O)CH2CH2C(O)S-, - NHC(O)CH2CH2C(O)S-, -OC(O)CH2C(O)O-, -NHC(O)CH2C(O)NH-, - NHC(O)CH2C(O)O-, -OC(O)CH2C(O)NH-, -OC(O)CH2C(O)S-, - NHC(O)CH2C(O)SH-, -OC(O)C(O)O-, -NHC(O)C(O)NH-, -NHC(O)C(O)O-, - OC(O)C(O)NH-, -OC(O)C(O)S-, -NHC(O)C(O)S-, -C(O)O-, -C(O)NH-, -C(O)S- - NH-, -O-, -S-, -S-S-, -C(N)H-O-, -NHC(O)O-, It may be a linker containing moietyes of -NHC(O)NH-, -OC(O)O-, and -OPO3-, or a linker containing a combination thereof. However, it is not limited to this.

The right end of the above moiety may represent a connection site with a compound of chemical formula I. The above moiety may include a spacer at the left end. The above spacers can be various spacers used in the industry for oligonucleotide synthesis, such as C1-30 alkyl, C2-30 alkenyl, and polyethylene glycol (PEG).

The above linker includes a spacer to secure a physical distance between the solid support and the compound of Formula I.

The above fixation method can be selectively adjusted according to specific synthesis conditions or goals and may include various bonding methods that stabilize the bond between the solid support and the compound of Formula I or impart functionality. However, this fixing method is not limited to this.

In the method for synthesizing oligonucleotides according to the present invention, the nucleoside phosphoamidite or nucleotide phosphoamidite may refer to a nucleoside or nucleotide derivatized with amidite. The above nucleoside or nucleotide is as described above.

The above nucleoside phosphamidite or nucleotide phosphamidite may be a modified nucleoside phosphamidite or a modified nucleotide phosphamidite.

The above modified nucleoside phosphoamidite or modified nucleotide phosphoamidite may have a modified sugar group. The variations of the group are as described above.

The above modified nucleoside phosphoamidite or modified nucleotide phosphoamidite may have a modified nucleobase. The modified nucleobase is as described above.

The above modified nucleoside phosphoamidite or modified nucleotide phosphoamidite may have a modified phosphate group. The modified phosphate group may be a phosphoamidite group derivatized into amidite. In addition, the modified phosphate group may include a phosphate ester group, a phosphorothioate ester group, or a phosphoramidate group formed by oxidizing, sulfating, or imiding the phosphoamidite group.

The above modified nucleoside phosphoamidite or modified nucleotide phosphoamidite may have a protecting group attached to a phosphorus residue. The above-mentioned protecting group may be a methyl group or a cyanoethyl group.

In one embodiment, the modified nucleoside phosphoamidite or modified nucleotide phosphoamidite may be a 2'-modified nucleoside phosphoamidite or nucleotide phosphoamidite, or a 2'-substituted nucleoside phosphoamidite or nucleotide phosphoamidite. Specifically, the modified nucleoside phosphoamidite or modified nucleotide phosphoamidite may be a nucleoside phosphoamidite or nucleotide phosphoamidite modified or substituted with 2'-O-alkyl, 2'-O-methyl, 2'-O-ethyl, 2'-fluoro, 2'-O-methoxyethyl, 2'-amino, 2'-arabino-fluoro, 2'-O-aryl, 2'-O-benzyl, 2'-O-allyl, 2'-acetyl, 2'-aminomethyl, 2'-thio, or 2'-O-methyl-4-pyridine.

In one embodiment, the modified nucleoside phosphoamidite or the modified nucleotide phosphoamidite may have a hydroxyl group (-OH) at the 5' or 3' position substituted with a thiol group (-SH) or an amino group (-NH2).

In one embodiment, the modified nucleoside phosphamidite or the modified nucleotide phosphamidite may be an abasic nucleoside phosphamidite or an abasic nucleotide phosphamidite. Abasic nucleoside phosphoamidite or abasic nucleotide phosphoamidite means a nucleoside phosphoamidite or nucleotide phosphoamidite without attached nucleobases.

In one embodiment, the modified nucleoside phosphoamidite or the modified nucleotide phosphoamidite may be an inverted nucleoside phosphoamidite or an inverted nucleotide phosphoamidite. Inverted nucleoside phosphoamidite or inverted nucleotide phosphoamidite means a nucleoside phosphoamidite or nucleotide phosphoamidite having a structure in which the binding direction is opposite to that of a normal nucleoside phosphoamidite or nucleotide phosphoamidite. For example, in a method for synthesizing oligonucleotides on a solid support, a normal nucleoside phosphoamidite or nucleotide phosphoamidite is connected to the next monomer through a functional group at the 5' position, but an inverted nucleoside phosphoamidite or inverted nucleotide phosphoamidite is connected to the next monomer through a functional group at the 3' position.

In one embodiment, the modified nucleoside phosphoamidite or the modified nucleotide phosphoamidite may be an inverted abasic nucleoside phosphoamidite or an inverted abasic nucleotide phosphoamidite.

In one embodiment, the nucleotide phosphoamidite may be a nucleotide phosphoamidite, a di-nucleotide phosphoamidite, a tri-nucleotide phosphoamidite, or a nucleotide phosphoamidite of an oligomer with more than one of these.

In the method for synthesizing an oligonucleotide according to the present invention, the nucleoside phosphoamidite or nucleotide phosphoamidite may have a phosphoamidite group at the 3' or 5' position by derivatizing any one of the hydroxyl group, thiol group, or amino group at the 3' position, or the hydroxyl group, thiol group, or amino group at the 5' position into an amidite group. When a nucleoside phosphoamidite or nucleotide phosphoamidite has a phosphoamidite group at the 3' position, a protecting group may be attached at the 5' position, and when a nucleoside phosphoamidite has a phosphoamidite group at the 5' position, a protecting group may be attached at the 3' position. The 5' position, 3' position, and protector are as described above.

In one embodiment, the nucleoside phosphoamidite or nucleotide phosphoamidite may be a monomer, a dimer, a trimer, or more oligomers.

In the method for synthesizing oligonucleotides according to the present invention, the step (ii)
A deprotected hydroxyl, thiol, or amino group at the 5' position is connected to the 3' position of a nucleoside phosphoamidite or nucleotide phosphoamidite having a protecting group on the hydroxyl, thiol, or amino group at the 5' position;
A deprotected hydroxyl, thiol, or amino group at the 5' position is connected to the 5' position of a nucleoside phosphoamidite or nucleotide phosphoamidite having a protecting group on a hydroxyl, thiol, or amino group at the 3' position;
A deprotected hydroxyl, thiol, or amino group at the 3' position is connected to the 3' position of a nucleoside phosphoamidite or nucleotide phosphoamidite having a protecting group on a hydroxyl, thiol, or amino group at the 5' position;
A deprotected hydroxyl, thiol, or amino group at the 3' position may be connected to the 5' position of a nucleoside phosphoamidite or nucleotide phosphoamidite having a protecting group on the hydroxyl, thiol, or amino group at the 3' position.

In one embodiment, the nucleoside phosphoamidite or nucleotide phosphoamidite having a protecting group on a hydroxyl group, thiol group, or amino group at the 5' or 3' position may be an abasic nucleoside phosphoamidite or an abasic nucleotide phosphoamidite.

In one embodiment, the nucleoside phosphoamidite or nucleotide phosphoamidite having a protecting group on the hydroxyl group, thiol group, or amino group at the 3' position may mean an inverted nucleoside phosphoamidite or an inverted nucleotide phosphoamidite.

In one embodiment, the nucleoside phosphoamidite or nucleotide phosphoamidite having a protecting group on the hydroxyl group, thiol group, or amino group at the 3' position may be an inverted abasic nucleoside phosphoamidite or an inverted abasic nucleotide phosphoamidite.

In the method for synthesizing an oligonucleotide according to the present invention, step (ii) of the last iteration may be a step of coupling the 5' position of a nucleoside phosphoamidite or nucleotide phosphoamidite having a protecting group on the hydroxyl group, thiol group, or amino group at the 3' position to the hydroxyl group, thiol group, or amino group at the 3' position of a deprotected 5' position or 3' position.

In one embodiment, the nucleoside phosphoamidite or nucleotide phosphoamidite having a protecting group on the hydroxyl group, thiol group, or amino group at the 3' position may be an abasic nucleoside phosphoamidite or an abasic nucleotide phosphoamidite.

In one embodiment, the nucleoside phosphoamidite or nucleotide phosphoamidite having a protecting group on the hydroxyl group, thiol group, or amino group at the 3' position may mean an inverted nucleoside phosphoamidite or an inverted nucleotide phosphoamidite.

In one embodiment, the nucleoside phosphoamidite or nucleotide phosphoamidite having a protecting group on the hydroxyl group, thiol group, or amino group at the 3' position may be an inverted abasic nucleoside phosphoamidite or an inverted abasic nucleotide phosphoamidite.

In the method for synthesizing an oligonucleotide according to the present invention, step (vi) may be a step of synthesizing a desired oligonucleotide by repeating steps (ii) to (v) 1 to 100 times, 1 to 75 times, or 1 to 50 times.

In one specific example, the last step of the last iteration may be step (iii) or (iv).

In the method for synthesizing oligonucleotides according to the present invention, one or more of the steps (i) to (v) may be performed at a temperature below room temperature (RT). Specifically, steps (i), (ii), (iii), (iv), one or more of steps (i) to (v), one or more of steps (i) to (iv), one or more of steps (ii) to (iii), steps (i), (ii) and (iv), steps (i), (iii) and (iv), steps (i) and (iv), steps (i), (ii), (iii) and (iv), steps (i), (ii) and (iv), or steps (ii) and (iii) may be performed at a temperature below room temperature. More specifically, step (ii), steps (i), (ii), and (iv), or steps (i) through (iv) may be performed at a temperature below room temperature. Additionally, the step of capping a deprotected hydroxyl group, thiol group, or amino group that has not undergone the coupling reaction may be performed at a temperature below room temperature.

In one embodiment, the temperature may refer to an inlet temperature.

In the oligonucleotide synthesis method according to the present invention, the temperature below room temperature (RT) may be less than 22 °C. Specifically, temperatures below room temperature (RT) are less than 22 °C, less than 21 °C, 20 °C or lower, 0 °C or higher and less than 22 °C, 5 °C or higher and less than 22 °C, 10 °C or higher and less than 22 °C, 12 °C or higher and less than 22 °C, 13 °C or higher and less than 22 °C, 15 °C or higher and less than 22 °C, 0 °C or higher and 20 °C or lower, 5 °C or higher and 20 °C or lower, 10 °C or higher and 20 °C or lower, 12 °C or higher and 20 °C or lower, 13 °C or higher and 20 °C or lower, 15 °C or higher and 20 °C or lower, 0 °C or higher and 19 °C or lower, 5 °C or higher and 19 °C or lower, 10 °C or higher and 19 °C or lower, 12 °C or higher and 19 °C or lower, and 13 °C or higher and 19 It may be °C or lower, 15 °C or higher and 19 °C or lower, 0 °C or higher and 18 °C or lower, 5 °C or higher and 18 °C or lower, 10 °C or higher and 18 °C or lower, 12 °C or higher and 18 °C or lower, 13 °C or higher and 18 °C or lower, or 15 °C or higher and 18 °C or lower.

That is, the oligonucleotide synthesis method according to the present invention comprises one or more of steps (i), (ii), (iii), (iv), and (v), one or more of steps (i) to (iv), one or more of steps (ii) to (iii), steps (i), (ii) and (iv), steps (i), (iii) and (iv), steps (i) and (iv), steps (i), (ii), (iii) and (iv), steps (i), (ii) and (iv), or steps (ii) and (iii), specifically, step (ii), steps (i), (ii), and (iv), or steps (i) to (iv) are less than 22°C, less than 21°C, 20°C or less, 0°C or more and less than 22°C, 5°C or more and less than 22°C, 10°C or more and less than 22°C, 12°C or more and less than 22°C, 13°C or more Less than 22 °C, 15 °C or more and less than 22 °C, 0 °C or more and 20 °C or less, 5 °C or more and 20 °C or less, 10 °C or more and 20 °C or less, 12 °C or more and 20 °C or less, 13 °C or more and 20 °C or less, 15 °C or more and 20 °C or less, 0 °C or more and 19 °C or less, 5 °C or more and 19 °C or less, 10 °C or more and 19 °C or less, 12 °C or more and 19 °C or less, 13 °C or more and 19 °C or less, 15 °C or more and 19 °C or less, 0 °C or more and 18 °C or less, 5 °C or more and 18 °C or less, 10 °C or more and 18 °C or less, 12 °C or more and 18 °C or less, 13 °C or more and 18 °C or less, or 15 °C or more and 18 It may be performed at a temperature of °C or lower.

### Effects of Invention

The oligonucleotide synthesis method according to the present invention can stably synthesize oligonucleotides with higher purity by carrying out the synthesis cycle at a temperature below room temperature, thereby suppressing side reactions occurring during the synthesis process.

### Brief Description of Drawings

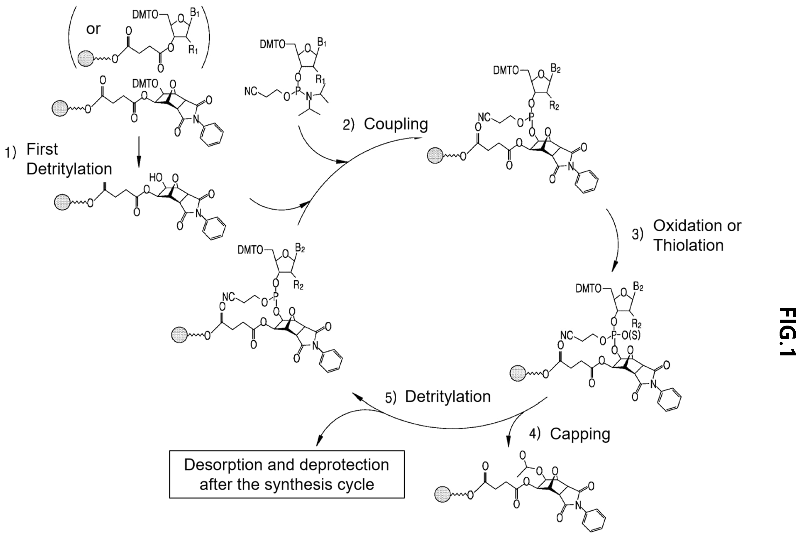

Figure 1 is a schematic diagram of an oligonucleotide synthesis method.

### Mode for Carrying Out the Invention

Hereinafter, preferred embodiments are presented to aid in understanding the present invention. However, the following examples are provided merely to facilitate a better understanding of the present invention, and the content of the present invention is not limited by the following examples. Since the embodiments are capable of various modifications, the embodiments are not limited to the embodiments disclosed below but can be implemented in various forms.

Terms or words used in the specification and claims of the present invention shall not be interpreted as being limited to their ordinary or dictionary meanings, and shall be interpreted in a meaning and concept consistent with the technical spirit of the present invention, based on the principle that the inventor may appropriately define the concept of the terms to best describe his invention.

Throughout the specification of the present invention, when a part is described as "comprising" a certain component, this means that, unless specifically stated otherwise, it does not exclude other components but may include additional components.

Throughout the specification of the present invention, "A and/or B" means A or B, or A and B.

### Example 1. Synthesis of oligonucleotides

Oligonucleotides are solid supports to which inverted baseless nucleosides are attached (Manufacturer: Kinovate, loading capacity: It was synthesized on a scale of 1-3 mmol using an AKTA oligopilot 100 solid-phase synthesizer (300 µmol/g).

The structure of the solid support to which the above-mentioned inverted basic nucleoside is attached is as shown in Chemical Formula II below.

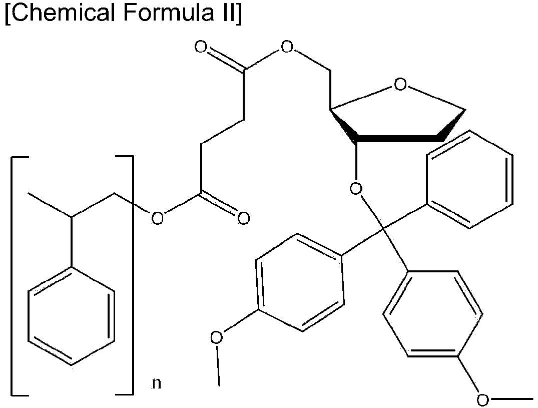

Specifically, the method of synthesizing an oligonucleotide by linking individual nucleosides according to the desired sequence consists of the following four-step synthesis cycle (Fig. 1):
1) Detritylation
2) Coupling
3) Oxidation or Thiolation
4) Capping.

Nucleoside phosphoamidite, all other synthetic reagents, and solvents were obtained from commercially available sources and prepared and used according to the reagent composition without any purification or processing. For example, nucleoside phosphoamidite can be obtained from Hongene Biotech, Thermo, and sigma, dichloroacetic acid from CABB, and acetonitrile and toluene from SK chemical.

The detritylation step was performed using 2.5-10% dichloroacetic acid in toluene as a reagent.

The coupling step was performed by circulating a solution of nucleoside-phosphoramidite (0.2 M in acetonitrile) and 5-Ethylthio-1H-Tetrazole (0.5-1.0 M in acetonitrile) corresponding to the desired oligonucleotide sequence through a solid support.

The oxidation step was performed using pyridine and 0.05 M iodine in water. The thiolation step was performed using 0.2 M 3-amino-1,2,4-dithiazole-5-thion (XH) in pyridine.

The capping step was performed using a mixture of acetosan anhydride, 2,6-rutidine, and N-methylimidazole in acetonitrile.

Deprotection (removal of cyanoethyl protecting group) of cyanoethyl phosphodiester was performed using a 20% diethylamine (DEA) solution in acetonitrile (AN).

After the synthesis of solid-phase oligonucleotides was completed, the desorption and deprotection (removal of protecting groups from bases) of the oligonucleotides from the solid support were performed by treating with 28-30% ammonium hydroxide at 40-60°C for 6-20 hours.

The composite parameters are summarized in Table 1 below.

**[Table 1]**

| Processing steps | Variability | Reaction-cycle | unit |
|---|---|---|---|
| Composite support | Support | Solid support with inverted baseless nucleosides attached | - |
| | Load | 300~350 | µmol/g |
| Detritilization | reagent | 2.5 - 10% DCA in Toluene | |
| | Removal volume | 4.3 - 11.7 | CV |
| | Reaction time | 4.5 - 12.3 | minute |
| coupling | Nucleoside phosphoamidite equivalents | 1.5 -1.7 | eq |
| | Nucleoside phosphoamidite concentration | 0.2 | M |
| | Activator: Nucleoside phosphoamidite B | 3 : 2 | - |
| | Activator | 0.5 - 1.0M ONE | - |
| | Recirculation flow rate | 100-200 | cm/hr |
| | Recirculation time | 7.0 | minute |
| oxidation | reagent | 0.05 M I₂ in PYD/H2O | - |
| | Charging volume | 2.1 | CV |
| | Reaction time | 4.3 | minute |
| sulfuration | reagent | 0.1 M XH in PYD/AN | - |
| | Charging volume | 2.2 | CV |
| | Reaction time | 4.6 | minute |
| Capping | reagent | Cap A, 2:8 [NMI/AN] | - |
| | | Cap B 2:3:5 [Ac₂O/2, 6-Lutidine/AN] | |
| | capping filling volume | 0.5 | CV |
| | Reaction time | 1.3 | minute |
| Cyanoethyl phosphodiester deprotection | reagent | 20% DEA in AN | - |
| | Charging volume | 4.8 | CV |
| | Reaction time | 10 | minute |

Oligonucleotides of the following preparation examples and comparative preparation examples were synthesized using this synthesis method.

In the oligonucleotide sequences of Preparation Examples 1 to 7 and Comparative Preparation Examples 1 to 16 synthesized below, the meanings of invAb, s, fa, fu, fc, and fg are as follows.

"invAb" is an inverted baseless nucleotide (nucleoside).

"s" is a phosphorothioate-nucleoside bond.

"fa, fu, fc, fg" are 2'-fluoro-modified nucleotides (nucleosides).

"a, c, g" are nucleotides (nucleosides) modified with 2'-OMe.

### Experimental Example 1. Analysis of purity and yield of synthesized oligonucleotides

The purity and yield of the oligonucleotide products of the synthesized preparation examples and comparative preparation examples were analyzed.

Specifically, purity was measured as UV purity (%) using the LC-UV method, and yield (%) was calculated by measuring the OD concentration using a UV spectrometer.

Specifically, UV purity (%) is HPLC (Manufacturer: Agilent, Model Name: Purity was measured according to the peak area of the UV signal within a retention time range of 5 to 30 minutes using a 1290 infinity (1260 DAD) instrument. OD concentration is measured using a UV/Vis Spectrophotometer (Manufacturer: Agilent, Model Name: Absorbance was measured at a wavelength of 260 nm using Cary-3500.

### Preparation Example 1.

According to the method of Example 1, the oligonucleotide of Preparation Example 1, a 23-mer represented by the following sequence, was prepared. The average temperature of the above 4-step synthesis cycle process of Preparation Example 1 is 17.0 °C.

Sequence of Preparation Example 1 (Sequence No. 1): 5'-**asguggcu uafufcfcuauuucaucus** invAb-3'.

The UV purity of Preparation Example 1 was found to be significantly high at 90.7%, and the yield was found to be 80.1%.

### Comparative Preparation Example 1.

According to the method of Example 1, the oligonucleotide of Comparative Preparation Example 1 of a 23-mer represented by the following sequence was prepared. The average temperature of the above 4-step synthesis cycle process of Comparative Preparation Example 1 is 23.2°C.

Sequence of Comparative Preparation Example 1 (Sequence No. 1): 5'-**asguggcuuafufcfcuauuucaucus** invAb-3'.

The UV purity of Comparative Preparation Example 1 was found to be 88.5%, and the yield was found to be 79.8%.

### Preparation Example 2.

According to the method of Example 1, the oligonucleotide of Preparation Example 2, a 24-mer represented by the following sequence, was prepared. The average temperature of the above 4-step synthesis cycle process of Preparation Example 2 is 16.5°C.

Sequence of Preparation Example 2 (Sequence No. 2): 5'-**invAbsagug gcuuafufcfcuauuucaucus** invAb-3'.

The UV purity of Preparation Example 2 was found to be significantly high at 91.4%, and the yield was 74.6%.

### Comparative Preparation Example 2.

According to the method of Example 1, the oligonucleotide of Comparative Preparation Example 2, a 24-mer represented by the following sequence, was prepared. The average temperature of the above 4-step synthesis cycle process of Comparative Preparation Example 2 is 23.1°C.

Sequence of Comparative Preparation Example 2 (Sequence No. 2): 5'-invAbs a g u g g c u u a fu fc fc u a u u u c a u c us invAb-3'.

The UV purity of Comparative Preparation Example 2 was 88.2%, and the yield was 72.9%.

### Comparative Preparation Example 3.

According to the method of Example 1, the oligonucleotide of Comparative Preparation Example 3, a 24-mer represented by the following sequence, was prepared. The average temperature of the first cycle process in the above 4-step synthesis cycle process of Comparative Preparation Example 3 is 17.3°C, and the average temperature of the second through last cycles is 23.6°C.

Sequence of Comparative Preparation Example 3 (Sequence No. 2): 5'-invAbs a g u g g c u u a fu fc fc u a u u u c a u c us invAb-3'.

The UV purity of Comparative Preparation Example 3 was found to be 88.5%, and the yield was found to be 69.2%.

### Comparative Preparation Example 4.

According to the method of Example 1, the oligonucleotide of Comparative Preparation Example 4, a 24-mer represented by the following sequence, was prepared. The average temperature of the first to 13th cycle processes in the above 4-step synthesis cycle process of Comparative Preparation Example 4 is 23.6 °C, and the average temperature of the 14th to last cycle processes is 17.1 °C.

Sequence of Comparative Preparation Example 4 (Sequence No. 2): 5'-invAbs a g u g g c u u a fu fc fc u a u u u c a u c us invAb-3'.

The UV purity of Comparative Preparation Example 4 was 89.7%, and the yield was 70.1%.

### Preparation Example 3.

According to the method of Example 1, the oligonucleotide of Preparation Example 3, a 24-mer represented by the following sequence, was prepared. The average temperature of the above 4-step synthesis cycle process of Preparation Example 3 is 17.1°C.

Sequence of Preparation Example 3 (Sequence No. 3): 5'-invAbs a g u invAb**gcuuafufcfcuauuucaucu**s invAb-3'.

The UV purity of Preparation Example 3 was found to be significantly high at 90.9%, and the yield was 70.1%.

### Comparative Preparation Example 5.

According to the method of Example 1, the oligonucleotide of Comparative Preparation Example 5, a 24-mer represented by the following sequence, was prepared. The average temperature of the above 4-step synthesis cycle process of Comparative Preparation Example 5 is 23.4°C.

Sequence of Comparative Preparation Example 5 (Sequence No. 3): 5'-invAbs**aguinvAbgcuuafufcfcuauuucaucu**s invAb-3'.

The UV purity of Comparative Preparation Example 5 was 88.3%, and the yield was 68.8%.

### Comparative Preparation Example 6.

According to the method of Example 1, the oligonucleotide of Comparative Preparation Example 6, a 23-mer represented by the following sequence, was prepared. The solid support is a solid support with 2'-OMe Adenosine (Bz, with a benzoyl protecting group) attached (Manufacturer: Kinovate, loading capacity: 350 µmol/g) was used. The average temperature of the above 4-step synthesis cycle process of Comparative Preparation Example 6 is 17.2°C.

Sequence of Comparative Preparation Example 6 (Sequence No. 4): 5'-**asguggcuuafufcfcuauuucaucusa**-3'.

The UV purity of Comparative Preparation Example 6 was found to be 85.6%, and the yield was found to be 75.8%.

### Comparative Preparation Example 7.

According to the method of Example 1, the oligonucleotide of Comparative Preparation Example 7, a 23-mer represented by the following sequence, was prepared. The solid support is a solid support with 2'-O-Me adenosine attached (Manufacturer: Kinovate, loading capacity: 350 µmol/g) was used. The average temperature of the above 4-step synthesis cycle process of Comparative Preparation Example 7 is 15.9 °C.

Sequence of Comparative Preparation Example 7 (Sequence No. 4): 5'-**asguggcuuafufcfcuauuucaucus**a-3'.

The UV purity of Comparative Preparation Example 7 was 83.8%, and the yield was 74.6%.

### Comparative Preparation Example 8.

According to the method of Example 1, the oligonucleotide of Comparative Preparation Example 8, a 23-mer represented by the following sequence, was prepared. The solid support is a solid support with 2'-O-Me adenosine attached (Manufacturer: Kinovate, loading capacity: 350 µmol/g) was used. The average temperature of the above 4-step synthesis cycle process of Comparative Preparation Example 8 is 22.7°C.

Sequence of Comparative Preparation Example 8 (Sequence No. 4): 5'-**asguggcuuafufcfcuauuucaucusa**-3'.

The UV purity of Comparative Preparation Example 8 was found to be 85.6%, and the yield was found to be 75.8%.

### Comparative Preparation Example 9.

According to the method of Example 1, the oligonucleotide of Comparative Preparation Example 9, a 24-mer represented by the following sequence, was prepared. For the solid support, a solid support (CPG) with IdT (Inverted deoxy thymidine) attached was used. The average temperature of the above 4-step synthesis cycle process of Comparative Preparation Example 9 is 17.5°C.

Sequence of Comparative Preparation Example 9 (Sequence No. 5): 5'-invAbs a g u g g c u u a fu fc fc u a u u u c a u c us IdT-3'.

The UV purity of Comparative Preparation Example 9 was 81.6%, and the yield was 54.9%.

### Comparative Preparation Example 10.

According to the method of Example 1, the oligonucleotide of Comparative Preparation Example 10, a 24-mer represented by the following sequence, was prepared. For the solid support, a solid support (CPG) with IdT (Inverted deoxy thymidine) attached was used. The average temperature of the above 4-step synthesis cycle process of Comparative Preparation Example 10 is 23.8°C.

Sequence of Comparative Preparation Example 10 (Sequence No. 5): 5'-invAbs a g u g g c u u a fu fc fc u a u u u c a u c us IdT-3'.

The UV purity of Comparative Preparation Example 10 was 82.8%, and the yield was 55.7%.

### Comparative Preparation Example 11.

According to the method of Example 1, the oligonucleotide of Comparative Preparation Example 11, a 24-mer represented by the following sequence, was prepared. The solid support is a solid support with 2'-OMe uridine attached (Manufacturer: Kinovate, loading capacity: 350 µmol/g) was used. The average temperature of the coupling step during the above 4-step synthesis cycle process of Comparative Preparation Example 11 is 17.6°C, and the average temperature of the other three steps is 23.1 °C.

Sequence of Comparative Preparation Example 11 (Sequence No. 6): 5'-invAbs a g u g g c u u a fu fc fc u a u u u c a u c invAbs u-3'.

The UV purity of Comparative Preparation Example 11 was 83.6%, and the yield was 69.0%.

### Comparative Preparation Example 12.

According to the method of Example 1, the oligonucleotide of Comparative Preparation Example 12, a 24-mer represented by the following sequence, was prepared. The solid support is a solid support with 2'-OMe uridine attached (Manufacturer: Kinovate, loading capacity: 350 µmol/g) was used. The average temperature of the above 4-step synthesis cycle process of Comparative Preparation Example 12 is 23.1°C.

Sequence of Comparative Preparation Example 12 (Sequence No. 6): 5'-invAbs**aguggcuuafufcfcuauuucauc** invAbs u-3'.

The UV purity of Comparative Preparation Example 12 was 85.3%, and the yield was 68.1%.

### Comparative Preparation Example 13.

According to the method of Example 1, the oligonucleotide of Comparative Preparation Example 13, a 24-mer represented by the following sequence, was prepared. The average temperature of the above 4-step synthesis cycle process of Comparative Preparation Example 13 is 22.9 °C.

Sequence of Comparative Preparation Example 13 (Sequence No. 2): 5'-invAbs**aguggcuuafufcfcuauuucaucu**s invAb-3'.

The UV purity of Comparative Preparation Example 13 was 86.0%, and the yield was 66.2%.

### Comparative Preparation Example 14.

According to the method of Example 1, the oligonucleotide of Comparative Preparation Example 14, a 24-mer represented by the following sequence, was prepared. The average temperature of the oxidation/sulfidation step during the above 4-step synthesis cycle process of Comparative Preparation Example 14 is 16.5 °C / 17.1 °C, and the average temperature of the other three steps is 22.9 °C.

Sequence of Comparative Preparation Example 14 (Sequence No. 2): 5'-invAbs**aguggcuuafufcfcuauuucaucus** invAb-3'.

The UV purity of Comparative Preparation Example 14 was 85.6%, and the yield was 64.3%.

### Comparative Preparation Example 15.

According to the method of Example 1, the oligonucleotide of Comparative Preparation Example 15, a 24-mer represented by the following sequence, was prepared. The average temperatures of the detritylation and coupling steps during the above 4-step synthesis cycle process of Comparative Preparation Example 15 are 17.6°C and 18.5°C, and the average temperatures of the other two steps are 22.9°C.

Sequence of Comparative Preparation Example 15 (Sequence No. 2): 5'-invAbs**aguggcuuafufcfcuauuucaucu**s invAb-3'.

The UV purity of Comparative Preparation Example 15 was 89.1%, and the yield was 68.1%.

### Preparation Example 4.

According to the method of Example 1, the oligonucleotide of Preparation Example 4, a 24-mer represented by the following sequence, was prepared. The average temperature of the coupling step in the above 4-step synthesis cycle process of Preparation Example 4 is 17.4 °C, and the average temperature of the other three steps is 22.9 °C.

Sequence of Preparation Example 4 (Sequence No. 2): 5'-invAbs **agug gcuuafufcfcuauuucaucus** invAb-3'.

The UV purity of Preparation Example 4 was found to be significantly high at 90.8%, and the yield was 66.0%.

### Preparation Example 5.

According to the method of Example 1, the oligonucleotide of Preparation Example 5, a 24-mer represented by the following sequence, was prepared. The average temperature of the coupling step in the above 4-step synthesis cycle process of Preparation Example 5 is 19.0 °C, and the average temperature of the other three steps is 22.9 °C.

Sequence of Preparation Example 5 (Sequence No. 2): 5'-invAbs **agug gcuuafufcfcuauuucaucu**s invAb-3'.

The UV purity of Preparation Example 5 was found to be significantly high at 90.1%, and the yield was 66.8%.

### Preparation Example 6.

According to the method of Example 1, the oligonucleotide of Preparation Example 6, a 24-mer represented by the following sequence, was prepared. The average temperature of the coupling step during the above 4-step synthesis cycle process of Preparation Example 6 is 15.1°C, and the average temperature of the other three steps is 22.9°C.

Sequence of Preparation Example 6 (Sequence No. 2): 5'-invAbs **agug gcuuafufcfcuauuucaucu**s invAb-3'.

The UV purity of Preparation Example 6 was found to be significantly high at 90.7%, and the yield was 66.0%.

### Preparation Example 7.

According to the method of Example 1, the oligonucleotide of Preparation Example 7, a 24-mer represented by the following sequence, was prepared. The average temperature of the coupling step in the above 4-step synthesis cycle process of Preparation Example 7 is 13.0 °C, and the average temperature of the other three steps is 22.9 °C.

Sequence of Preparation Example 7 (Sequence No. 2): 5'-invAbs **agug gcuuafufcfcuauuucaucus** invAb-3'.

The UV purity of Preparation Example 7 was found to be significantly high at 90.6%, and the yield was 67.5%.

### Comparative Preparation Example 16.

According to the method of Example 1, the oligonucleotide of Comparative Preparation Example 16, a 24-mer represented by the following sequence, was prepared. The average temperature of the coupling step during the above 4-step synthesis cycle process of Comparative Preparation Example 16 is 10.9°C, and the average temperature of the other three steps is 22.9°C.

Sequence of Comparative Preparation Example 16 (Sequence No. 2): 5'-invAbs**aguggcuuafufcfcuauuucaucu**s invAb-3'.

The UV purity of Comparative Preparation Example 16 was 86.9%, and the yield was 65.6%.

### Comparison of Purity and Yield of Synthesized Oligonucleotides

The process temperature, UV purity, and yield of the oligonucleotides of Preparation Examples 1 to 7 and Comparative Preparation Examples 1 to 16 were compared and are shown in Table 2 below.

**[Table 2]**

| | Control step | Ave temp (°C) | UV Purity (%) | Yield (%) |
|---|---|---|---|---|
| Preparation Example 1 | All processes | 17.0 | 90.7 | 80.1 |
| Comparative Preparation Example 1 | All processes | 23.2 | 88.5 | 79.8 |
| Preparation Example 2 | All processes | 16.5 | 91.4 | 74.6 |
| Comparative Preparation Example 2 | All processes | 23.1 | 88.2 | 72.9 |
| Comparative Preparation Example 3 | Cycle 1^{st} /Cycle 2^{nd} - end | 17.3 / 23.6 | 88.5 | 69.2 |
| Comparative Preparation Example 4 | Cycle 1^{st} - 13^{th} /Cycle 14^{th} - end | 23.6 / 17.1 | 89.7 | 70.1 |
| Preparation Example 3 | All processes | 17.1 | 90.9 | 70.1 |
| Comparative Preparation Example 5 | All processes | 23.4 | 88.3 | 68.8 |
| Comparative Preparation Example 6 | All processes | 17.2 | 85.6 | 75.8 |
| Comparative Preparation Example 7 | All processes | 15.9 | 83.8 | 74.6 |
| Comparative Preparation Example 8 | All processes | 22.7 | 85.6 | 75.8 |
| Comparative Preparation Example 9 | All processes | 17.5 | 81.6 | 54.9 |
| Comparative Preparation Example 10 | All processes | 23.8 | 82.8 | 55.7 |
| Comparative Preparation Example 11 | Coupling / The rest | 17.6 / 23.1 | 83.6 | 69.0 |
| Comparative Preparation Example 12 | All processes | 23.1 | 85.3 | 68.1 |
| Comparative Preparation Example 13 | All processes | 22.9 | 86.0 | 66.2 |
| Comparative Preparation Example 14 | Oxidation, sulfidation / remain | 16.5, 17.1 / 22.9 | 85.6 | 64.3 |
| Comparative Preparation Example 15 | Detritylation, coupling / remain | 17.6, 18.5 / 22.9 | 89.1 | 68.1 |
| Preparation Example 4 | Coupling / The rest | 17.4 / 22.9 | 90.8 | 66.0 |
| Preparation Example 5 | Coupling / The rest | 19.0 / 22.9 | 90.1 | 66.8 |
| Preparation Example 6 | Coupling / The rest | 15.1 / 22.9 | 90.7 | 66.0 |
| Preparation Example 7 | Coupling / The rest | 13.0 / 22.9 | 90.6 | 67.5 |
| Comparative Preparation Example 16 | Coupling / The rest | 10.9 / 22.9 | 86.9 | 65.6 |

As shown in Table 2 above, it was confirmed that oligonucleotides have higher synthesis purity when synthesized at an average temperature lower than 22°C. These results were observed even when the sequence of oligonucleotide residues was changed using the same synthesis method. It was confirmed that solid-phase supports with inverted baseless nucleosides attached have higher synthetic purity. It was confirmed that controlling the average temperature of the entire synthesis process rather than controlling the temperature of each cycle results in higher synthesis purity. In addition, it was confirmed that higher synthesis purity is achieved when the average temperature of the coupling step process during the four reaction steps of the synthesis cycle is 22°C or lower.

From the above description, those skilled in the art to which the present invention pertains will understand that the present invention may be implemented in other specific forms without altering its technical concept or essential features. In this regard, the experimental examples and embodiments described above should be understood as exemplary in all respects and not limiting. The scope of the present invention should be interpreted as including all modifications or variations derived from the meaning and scope of the claims set forth below and equivalent concepts, rather than from the detailed description above.

## Claims

1. A method for synthesizing an oligonucleotide on a solid support comprising steps (i) to (v) below, wherein one or more of the steps are performed at a temperature below room temperature (RT):
(i) removing a protecting group from a nucleoside or nucleotide having a protecting group on a hydroxyl group, thiol group, or amino group at the 5' or 3' position to form a deprotected nucleoside or nucleotide having a hydroxyl group, thiol group, or amino group at the 5' or 3' position;
(ii) coupling a nucleoside phosphoamidite or nucleotide phosphoamidite having a protecting group on a hydroxyl group, thiol group, or amino group at the 5' or 3' position to a deprotected hydroxyl group, thiol group, or amino group at the 5' or 3' position;
(iii) oxidizing the phosphite ester group generated through coupling to form a phosphate ester group, thiolating to form a phosphorothioate ester group, or imizing to form a phosphoramidate group;
(iv) removing the protecting group of a hydroxyl group, thiol group, or amino group at the 5' or 3' position of a coupled nucleoside phosphoamidite or nucleotide phosphoamidite to form a deprotected hydroxyl group, thiol group, or amino group at the 5' or 3' position;
(v) synthesizing a desired oligonucleotide by repeating steps (ii) to (iv) one or more times.

2. The method according to claim 1, further comprising the step of capping a deprotected hydroxyl group, a thiol group, or an amino group that has not undergone a coupling reaction.

3. The method according to claim 1, wherein, when steps (ii) to (iv) are repeated one or more times, in one or more of the repeated cycles, one or more of steps (ii) to (iv) are performed at a temperature below room temperature.

4. The method according to claim 1, wherein the last step of the last iteration is step (iii) or (iv).

5. The method according to claim 1, further comprising the step of removing a protecting group after step (v).

6. The method according to claim 1, wherein prior to step (i),
further comprising the step of preparing a nucleoside or nucleotide that is fixed to the surface of a solid support and has a protecting group on a hydroxyl group, a thiol group, or an amino group at the 5' or 3' position.

7. The method according to claim 1, further comprising the step of recovering the synthesized oligonucleotide.

8. The method according to claim 1, wherein the protecting group is trityl (Trt), dimethoxytrityl (DMT), monomethoxytrityl (MMT), tertbutyldimethylsilyl (TBDMS), trimethylsilyl (TMS), t-butoxycarbonyl (Boc), methyl phosphodiester, benzoyl (Bz), trifluoroacetyl (TFA), benzyl (Bn), p-methoxytriphenylmethyl (p-Methoxytrityl, PMT), mono-methoxyphosphodiester (MMP), fluorenylmethoxycarbonyl (Fmoc), tetrahydrofuran (THP), acetyl (Ac), mercaptotrityl A method for synthesizing an oligonucleotide having a protecting group of (mercapto-trityl, MTT), 4-methoxycitrate (Mmt), thiopyridyl (Thiopyridyl), benzylthio (Benzylthio), fluorenylmethoxycarbonyl (Fmoc), carbamate (Carbamate, Cbz), trifluoroacetyl (TFA), isobutyryl (iBu), phenoxyacetyl (PAC), or dimethylformamidine (dmf).

9. The method according to claim 1, wherein at step (i)
the nucleoside or nucleotide having a protecting group on a hydroxyl, thiol, or amino group at the 5' position is immobilized on the surface of a solid support through the 3' or 2' position; or
the nucleoside or nucleotide having a protecting group on a hydroxyl group, thiol group, or amino group at the 3' position is immobilized on the surface of a solid support through the 5' or 2' position.

10. The method according to claim 1, wherein at step (i),
the nucleoside or nucleotide having a protecting group on the hydroxyl, thiol, or amino group at the 3' position is an abasic nucleoside or nucleotide.

11. The method according to claim 10,
wherein the above-mentioned abasic nucleoside is an inverted abasic nucleoside represented by the following formula I, which is immobilized on the surface of a solid support via a linker: 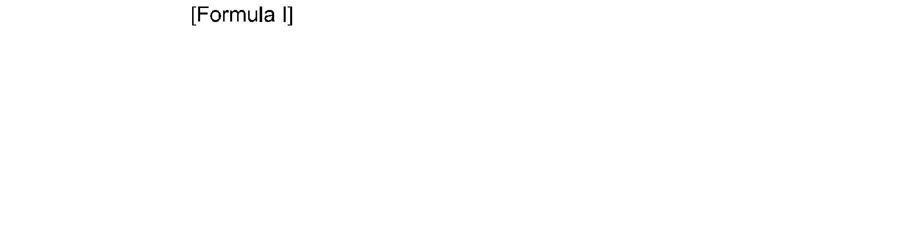 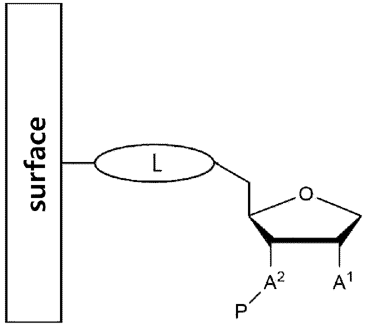
In the above equation, P is the protector and L is the linker, and A¹is -H, -OH, 2'-O-alkyl, 2'-O-methyl, 2'-O-ethyl, 2'-fluoro, 2'-O-methoxyethyl, 2'-amino, 2'-arabino-fluoro, 2'-O-aryl, 2'-O-benzyl, 2'-O-allyl, 2'-acetyl, 2'-aminomethyl, 2'-thio, or 2'-O-methyl-4-pyridine, and
A²is -O-, -NH-, or -S-.

12. The method according to claim 1, wherein (ii) step is
linking a deprotected hydroxyl, thiol, or amino group at the 5'-position to the 3'-position of a nucleoside phosphoramidite or nucleotide phosphoramidite having a protecting group on a hydroxyl, thiol, or amino group at the 5'-position;
linking a deprotected hydroxyl, thiol, or amino group at the 5'-position to the 5'-position of a nucleoside phosphoramidite or nucleotide phosphoramidite having a protecting group on a hydroxyl, thiol, or amino group at the 3'-position;
linking a deprotected hydroxyl, thiol, or amino group at the 3'-position to the 3'-position of a nucleoside phosphoramidite or nucleotide phosphoramidite having a protecting group on a hydroxyl, thiol, or amino group at the 5'-position; or
linking a deprotected hydroxyl, thiol, or amino group at the 3'-position to the 5'-position of a nucleoside phosphoramidite or nucleotide phosphoramidite having a protecting group on a hydroxyl, thiol, or amino group at the 3'-position.

13. The method according to claim 1,
wherein step (ii); steps (i), (ii), and (iv); or steps (i) to (iv) are performed at a temperature below room temperature.

14. The method according to any one of claims 1 to 13,
wherein the temperature below room temperature (RT) is less than 22 °C.
